# EUROPEAN PATENT SPECIFICATION

(11) **EP 2 548 549 B1**
(45) Date of publication and mention of the grant of the patent: **15.05.2019**
(21) Application number: 11755963.3
(22) Date of filing: 24.01.2011
(51) Int. Cl.: A61K 8/34, A61K 8/06, A61K 8/41, A61K 8/44, A61K 8/46, A61Q 19/00

(54) **EMULSION COMPOSITION COMPRISING A HIGHER ALIPHATIC ALCOHOL, AN ANIONIC SURFACTANT AND A CATIONIC SURFACTANT**
EMULSIONSZUSAMMENSETZUNG ENTHALTEND EIN HÖHERES ALIPHATISCHES ALKOHOL, EIN ANIONISCHES TENSID UND EIN KATIONISCHES TENSID
COMPOSITION DE TYPE ÉMULSION COMPRENANT UN ALCOOL ALIPHATIQUE SUPÉRIEUR, UN TENSIOACTIF ANIONIQUE ET UN TENSIOACTIF CATIONIQUE

(30) Priority: 27.04.2010 JP 2010102220; 17.03.2010 JP 2010060738
(43) Date of publication of application: 23.01.2013
(73) Proprietor: SHISEIDO COMPANY, LTD., Chuo-ku, Tokyo 104-8010 (JP)
(72) Inventor: UYAMA Makoto, Yokohama-shi Kanagawa 224-8558 (JP); KUROSAWA Takafumi, Yokohama-shi Kanagawa 224-8558 (JP); ISHIMATSU Takayuki, Yokohama-shi Kanagawa 224-8558 (JP)
(74) Representative: Ter Meer Steinmeister & Partner
(86) International application number: PCT/JP2011/051191
(87) International publication number: WO 2011/114773

(56) References cited:
- WO-A1-99/24004
- JP-A- 2003 201 224
- JP-A- 2008 231 035
- KR-A- 950 016 684
- US-A- 4 997 641
- US-A- 5 726 137

## Description

### TECHNICAL FIELD

The present invention relates to a non-therapeutic use of an emulsified composition as a skin care cosmetic. The skin care cosmetic is an emulsified composition involving *α*-gel wherein the viscosity of the emulsified composition does not decrease over time even when salts are not added.

### BACKGROUND ART

Generally, for emulsified compositions and gel compositions involving *α*-gel, the stability over time is one of the important elements; but a reduction in the viscosity of the emulsified composition is observed over time.

For example, Patent Document 1, an invention by the applicant of the present invention, discloses a gel-like composition and a cream composition that use *α*-gel composed of a higher alcohol, a long chain acyl sulfonate anionic surfactant, and water. For the stability of the viscosity of said composition over time, a technology that adds a salt of ascorbic acid 2-glucoside is established.

For example, Patent Document 2, an invention by the applicant of the present invention, discloses a specific cream composition that use *α*-gel composed of a higher alcohol having an average alkyl chain length of 18 or longer, a long chain acyl sulfonate anionic surfactant, and water. Said cream composition characteristically further contains a polar oil component having a molecular weight of 400 or less as an essential ingredient; in said composition, the polar oil component having a molecular weight of 400 or less is involved in the stability of the viscosity over time.

### {Prior art documents}

### {Patent Documents}

Patent Document 1: JP 2005-132808 A
Patent Document 2: JP 2008-44866 A

US2011/0105604 A1 discloses in [0016] an emulsified cosmetic comprising cetyl alcohol, sodium methyl cocoyl taurate, behentrimonium methosulfate, vegetable oil and water.

### SUMMARY OF INVENTION

### PROBLEM THAT INVENTION IS TO SOLVE

The present invention provides a new method to stabilize the viscosity of an emulsified composition involving *α*-gel composed of specific ingredients wherein the viscosity of the emulsified composition does not decrease over time even when salts from ascorbic acid 2-glucoside and such are not added. Its object is to provide a non-therapeutic use of an emulsified composition as a skin care cosmetic whose viscosity does not decrease over time and thus exhibits excellent stability over time.

### TECHNICAL SOLUTION

That is, the present invention provides a non-therapeutic use of an emulsified composition as a skin care cosmetic comprising the following (A)-(E) ingredients wherein the blend ratio of the (B) ingredient is 1-15 times the blend ratio of the (C) ingredient in terms of mole ratio, the pH of said emulsified composition is 3-11, and said emulsified composition is an emulsified cosmetic, wherein
the (A) ingredient is a higher aliphatic alcohol, the (B) ingredient is an anionic surfactant,
the (C) ingredient is a cationic surfactant, the (D) ingredient is water,
the (E) ingredient is an oil component,
characterized in that:
said (B) ingredient comprises a long chain acyl sulfonate anionic surfactant represented by the general formula (I),
   general formula (I)

   R₁CO-a-(CH₂)ₙSO₃M₁ (I)

   wherein in formula (I), R₁CO- denotes a saturated or unsaturated fatty acid residue (acyl group) having 10-22 carbon atoms on average; a denotes-NR₂-, wherein R₂ denotes a hydrogen atom or an alkyl group having 1-3 carbon atoms; M₁ denotes a hydrogen atom, alkali metal, alkali earth metal, ammonium, or organic amine; n denotes an integer 1-3,
said (C) ingredient, the cationic surfactant, comprises a dialkyl quaternary ammonium salt and/or monoalkyl quaternary ammonium salt.

### ADVANTAGEOUS EFFECTS

The present invention provides a non-therapeutic use of an emulsified composition as a skin care cosmetic wherein the viscosity of the emulsified composition does not decrease over time even when salts from ascorbic acid 2-glucoside and such are not added and thus the stability over time is excellent; this is accomplished by using a cationic compound in an emulsified composition having specific ingredients involving *α*-gel.

Also, the cationic compound for stabilizing the viscosity over time can manifest its effect with the blend ratio smaller than that for the salts such as ascorbic acid 2-glucoside.

Furthermore, the pronounced stability over time, i.e. no reduction in the viscosity over time, is an effect that is manifested over a wide pH range, not affected by the pH of said composition.

### MODE FOR CARRYING OUT THE INVENTION

Details of the emulsified composition used in the present invention are described below.

### <The (A) ingredient: A higher aliphatic alcohol>

In the emulsified composition, the (A) ingredient, the higher aliphatic alcohol, along with the (B) ingredient, the anionic surfactant, and the (D) ingredient, water, is a constituent ingredient of the *α*-gel.

The emulsified composition used in the present invention is an oil-in-water emulsified composition that has the *α*-gel as the outer phase and the (E) ingredient, the oil component, as the inner phase; it is an emulsified composition preferably used for creams, emulsions, etc.

Selection of the higher aliphatic alcohol used in the present invention is not limited as long as it can be used in the field of cosmetics. Examples of the saturated straight chain monovalent alcohol include dodecanol (= lauryl alcohol), tridecanol, tetradecanol (= myristyl alcohol), pentadecanol, hexadecanol (= cetyl alcohol), heptadecanol, octadecanol (= stearyl alcohol), nonadecanol, icosanol (= arachyl alcohol), henicosanol, docosanol (= behenyl alcohol), tricosanol, tetracosanol (= carnaubyl alcohol), pentacosanol, and hexacosanol (= ceryl alcohol). Examples of the unsaturated monovalent alcohol include elaidyl alcohol.

In the present invention, a saturated straight chain monovalent alcohol is preferable in terms of stability over time.
For the (A) ingredient, one, two or more types can be used. In the present invention, it is preferable to use a mixture of two or more aliphatic alcohols; even more preferable is a combination for which the melting point of the mixture is 60°C or higher. If this melting point is under 60°C, then, depending on the formulation, the temperature stability decreases and creaming may occur. In the present invention, a combination of stearyl alcohol and behenyl alcohol, for example, is preferable.

Also, the higher aliphatic alcohol preferably has an arithmetically derived average alkyl chain length of 18 or more. If the average chain length is less than 18, then the melting point of the emulsified composition of the present invention becomes lower and the high temperature stability may not be sufficient. The upper limit of the average alkyl chain length is not limited in particular; a preferable alkyl chain length is about 22.

### <Blend ratio>

In the present invention, the blend ratio of the higher aliphatic alcohol is preferably 0.1-20 wt%, more preferably 1.0-10 wt% relative to the total amount of the emulsified composition.

For the (A) ingredient, one, two or more types can be used concurrently.

### <The (B) ingredient: An anionic surfactant>

In the emulsified composition used in the present invention, the (B) ingredient, the anionic surfactant, along with the (A) ingredient, the higher aliphatic alcohol, and the (D) ingredient, water, is a constituent ingredient of the *α*-gel.

Examples of the anionic surfactants used in the present invention include fatty acid soaps (for example, sodium laurate and sodium palmitate); higher alkyl sulfuric ester salts (for example, sodium lauryl sulfate and potassium laurylsulfate); alkylether sulfuric ester salts (for example, POE-triethanolamine laurylsulfate and sodium POE-lauryl sulfate); N-acyl sarcosinic acids (for example, sodium N-lauroyl sarcosinate); higher fatty acid amide sulfonic acid salts (for example, sodium N-myristoyl N-methyl taurate, sodium cocoyl methyl taurate, and sodium laurylmethyl taurate); phosphoric ester salts (for example, sodium POE-oleyl ether phosphate and POE stearyl ether phosphoric acid); sulfosuccinates (for example sodium di-2-ethylhexylsulfosuccinate, sodium monolauroyl monoethanol amide polyoxyethylene sulfosuccinate, and sodium lauryl polypropylene glycol sulfosuccinate); alkyl benzene sulfonates (for example, sodium linear dodecyl benzene sulfonate, triethanolamine linear dodecyl benzene sulfonate, linear dodecyl benzene sulfonic acid); higher fatty acid ester sulfates (for example, hydrogenated coconut oil aliphatic acid glycerin sodium sulfate); N-acyl glutamates (for example, mono sodium N-lauroylglutamate, disodium N-stearoylglutamate, and sodium N-myristoyl-L-glutamate); POE-alkylether carboxylic acid; POE-alkylarylether carboxylate; *α*-olefin sulfonate; higher fatty acid ester sulfonates; sec-alcohol sulfates; higher fatty acid alkylol amide sulfates; sodium lauroyl monoethanolamine succinates; ditriethanolamine N-palmitoylaspartate; and sodium caseinate.

In the present invention, the (B) ingredient comprises a long chain acyl sulfonate anionic surfactant represented by the following general formula (I):

R₁CO-a-(CH₂)ₙSO₃M₁ (I)

In the general formula (I), R₁CO- denotes a saturated or unsaturated fatty acid residue (acyl group) having an average of 10-22 carbon atoms. Examples of R₁CO- include C₁₁H₂₃CO, C₁₂H₂₅CO, C₁₃H₂₇CO, C₁₄H₂₉CO, C₁₅H₃₁CO, C₁₆H₃₃CO, C₁₇H₃₅CO, coco-fatty acid residues, and palm-fatty acid residues. For R₁CO-, those having an average of 12-22 carbon atoms are more preferable from the point of view of safety and such.

"a" denotes - -NR₂-, where R₂ denotes a hydrogen atom or an alkyl group having 1-3 carbon atoms. These are electron donor groups. For a,-NH- or -N(CH₃)- are preferable.

M₁ represents a hydrogen atom, alkali metal, alkali earth metal, ammonium, or organic amine. Examples of M₁ include lithium, potassium, sodium, calcium, magnesium, ammonium, monoethanolamine, diethanolamine, triethanolamine, sodium taurate, and sodium N-methyltaurate.
n denotes an integer 1-3.

Examples of compounds for which a in the aforementioned general formula (1) denotes -NH-, i.e. a long chain acyl taurate cationic surfactant, include N-lauroyl taurate, N-cocoyl-N-ethanol taurate, N-myristoyl taurate, and N-stearoyl taurate.

Examples of compounds for which a in the aforementioned general formula (I) denotes -N(CH₃)-, i.e. a long chain acyl methyltaurate anionic surfactant, include N-lauroyl-N-methyltaurate, N-palmitoyl-N-methyltaurate, N-stearoyl-N-methyltaurate, and N-cocoyl-N-methyltaurate.

A particularly preferable specific long chain acyl sulfonate anionic surfactant is N-stearoyl-N-methyltaurate. More specifically, sodium N-stearoyl-N-methyltaurate is preferable.

On the other hand, the (B) ingredient can also comprise N-acyl-L-glutamate, although it is not a long chain acyl sulfonate anionic surfactant. More specifically, sodium N-acyl-L-glutamate can additionally be comprised as (B) ingredient.

### <Blend ratio>

In the present invention, the blend ratio of the anionic surfactant is preferably 0.01-2.0 wt% relative to the total amount of the emulsified composition. More preferably it is 0.1-1.5 wt%.

For the (B) ingredient, one, two or more types can be used concurrently.

Also, the blend ratio of the (B) ingredient must be 1-15 times the blend ratio of the (C) ingredient in terms of the mole ratio. Preferably, the blend ratio of the (B) ingredient/the blend ratio of the (C) ingredient should be 1-6 in terms of the mole ratio.

### <The (C) ingredient: A Cationic surfactant>

In the emulsified composition used in the present invention, the cationic surfactant is added to suppress the reduction in the viscosity of the emulsified composition of the present invention over time: it functions as a viscosity reduction suppressor.

Examples of the cationic surfactants used in the present invention include alkyltrimethylammonium salts (for example, stearyltrimethyl ammonium chloride and lauryltrimethyl ammonium chloride), alkylpyridinium salts (for example, cetylpyridinium chloride), distearyldimethylammonium chloride dialkyldimethylammonium salt; poly (N,N'-dimethyl-3,5-methylene piperidinium) chloride; alkyl quaternary ammonium salts; alkyl dimethylbenzyl ammonium salts; alkyl isoquinolinium salts; dialkylmorphonium salts; POE alkyl amines; alkyl amine salts; polyamine fatty acid derivatives; amylalcohol fatty acid derivatives; benzalkonium chloride; and benzethonium chloride.

The (C) ingredient comprises a dialkyl quaternary ammonium salt and/or a monoalkyl quaternary ammonium salt. More specifically, distearyl dimethyl ammonium chloride is the most preferable for the dialkyl quaternary ammonium salt. Also, alkyl trimethyl ammonium chloride solution is the most preferable for the monoalkyl quaternary ammonium salt. Specific examples include behenyl trimethyl ammonium chloride, lauryl trimethyl ammonium chloride, and stearyl trimethyl ammonium chloride; the most preferable is behenyl trimethyl ammonium chloride.

### <Blend ratio>

In the present invention, the blend ratio of the cationic surfactant is 0.01-1.2 wt%, more preferably 0.05-0.5 wt% relative to the total amount of the emulsified composition.

Whereas Patent Document 1 needs to have a high blend ratio of 2-5 wt% of ascorbic acid 2-glucoside, i.e. the salt that functions as a viscosity reduction suppressor, a small blend ratio of the (C) ingredient is sufficient, which is another point of inventiveness in the present invention.

For the (C) ingredient, one, two or more types can be used concurrently.

Also, in the present invention, as mentioned above, the blend ratio of the (B) ingredient must be 1-15 times the blend ratio of the (C) ingredient in terms of the mole ratio. Preferably, the blend ratio of the (B) ingredient/the blend ratio of the (C) ingredient should be 1-6 (mole ratio) when the (C) ingredient is a dialkyl quaternary ammonium salt; and the blend ratio of the (B) ingredient/the blend ratio of the (C) ingredient should be 1-4 (mole ratio) when the (C) ingredient is a monoalkyl quaternary ammonium salt.

### <The (D) ingredient: Water>

In the emulsified composition used in the present invention, water, along with the (A) ingredient, the higher aliphatic alcohol and the (B) ingredient, the anionic surfactant, is a constituent ingredient of the *α*-gel. In the emulsified composition of the present invention, water constitutes the water phase of the outer phase and the water component of the oil-in-water emulsified composition.

In the present invention, in the presence of water (the (D) ingredient), the (B) ingredient and the (A) ingredient form aggregates made of lamella-like bimolecular membranes, assuming a so-called *α*-gel state. Alpha-gel is a white, highly viscous gel obtained by dissolving a higher aliphatic alcohol and an anionic surfactant at a high temperature, followed by mixing with water and then cooling, or by melting a higher aliphatic alcohol at a high temperature, followed by mixing with an aqueous solution of an anionic surfactant and then cooling. In addition, the emulsified composition of the present invention can be prepared by mixing said *α*-gel and the (E) ingredient, the oil component, and following a conventional method.

The *α*-gel used in the present invention and the emulsified composition of the present invention must have a melting point of 60°C or higher, preferably 65°C or higher. A cream composition, particularly a cream composition used as a cosmetic, is required to maintain an unchanging state from low to high temperatures; it is particularly necessary to maintain stability at higher temperatures. The *α*-gel that constitutes the present invention has a melting point; its viscosity begins to decrease at the melting point for higher temperatures; after a long time in storage, ingredients having different specific gravities separate completely. "Sechiru Arukoru no Butsurikagaku (Physicochemistry of Cetyl Alcohol)" by Shoji Fukushima (Fragrance Journal, 1992) describes the melting point of *α*-gel as dependent on the type of the higher alcohol and the mole ratio between the higher alcohol and the surfactant.

That is, *α*-gel formed by a higher aliphatic alcohol, an anionic surfactant, and water normally exhibits a single melting point of the constituent higher aliphatic alcohol and the surfactant. The following is known: when the higher aliphatic alcohol/anionic surfactant ratio is small, the melting point of the *α*-gel gradually changes toward higher temperatures and the melting point stops changing when the higher aliphatic alcohol/anionic surfactant ratio reaches 3 : 1.

For measuring the melting point, a DSC (differential scanning calorimeter) can be used. FIG. 6 of Patent Document 1 shows changes in the melting point and the transition temperature, as measured with a DSC, of an *α*-gel sample as the mole ratio of behenyl alcohol and sodium N-stearoyl-N-methyltaurate is changed. In this figure, the horizontal axis shows the mole ratio of higher alcohol/sodium N-stearoyl-N-methyltaurate (SMT) and the vertical axis shows the temperature (°C). As clearly shown in this figure, the endothermic peak of a sample obtained by mixing both the higher alcohol and the surfactant with water is a single peak on the higher temperature side compared with the endothermic peak obtained with a sample of each of them separately dissolved or dispersed in water; when the higher alcohol ratio is small (the amount of the higher alcohol is small) the temperature of the endothermic peak of the aggregate is lower, and the temperature increases as the higher alcohol ratio increases; when the mole ratio is 3 : 1 the aggregate structure is completed and the melting point does not change until the mole ratio reaches 10 : 1; when the higher alcohol ratio increases beyond 10 : 1 and the higher alcohol becomes excessive (not involved in formation of *α*-gel) and the peak for the higher alcohol appears. At this stage the excess higher alcohol is known to be dispersed in the *α*-gel as fine crystals and playing an effective role in maintaining the viscosity. However, when it is in extreme excess, hydrated crystals of the higher alcohol grow and destroy the *α*-gel system, which is not desirable.

Based on the above findings, the desirable mole ratio of the (A) ingredient : the (B) ingredient in the *α*-gel and the emulsified composition of the present invention that uses it as the outer phase is 3 : 1 or more, i.e. the mole ratio (A)/(B) is preferably 3 or more, more preferably 4 : 1 or more, and most preferably 4 : 1 to 10 : 1. If the aforementioned mole ratio is less than 3 : 1, then the melting point of the *α-*gel has not risen enough and the high temperature stability is not sufficient. On the other hand, if it is over 10 : 1, then crystals of the coexisting higher alcohol crystals may grow and destabilize the *α*-gel, which is not desirable.

### <Blend ratio>

In the present invention, an acceptable blend ratio of water is 1 wt% or more relative to the total amount of the emulsified composition. The preferable blend ratio is 30-90 wt%, more preferable is 50-90 wt%.

### <The (E) ingredient: An Oil component>

The oil component used in the present invention is the oil component of the inner phase that constitutes the oil-in-water emulsified composition. The emulsified composition of the present invention can be prepared by adding the (E) ingredient, the oil component, to the constituent ingredients of the *α*-gel composition.

Examples of the preparation method include, but are not limited to: a method in which the (A) ingredient and the (B) ingredient are dissolved at a high temperature, which is then mixed with the (D) ingredient, which is cooled, to which the (C) ingredient is added and then the (E) ingredient is added; a method in which the (A) is dissolved at a high temperature, which is mixed with an aqueous solution of the (B) ingredient dissolved in the (D) ingredient, which is cooled, to which the (C) ingredient is added, to which the (E) ingredient is added; a method in which the (A) ingredient, the (C) ingredient, and the (E) ingredient are melted at a high temperature, which is mixed with an aqueous solution of the (B) ingredient dissolved in the (D) ingredient, followed by cooling; a method in which the (A) ingredient and the (E) ingredient are melted at a high temperature, which is mixed with an aqueous solution of the (B) ingredient and the (C) ingredient dissolved in the (D) ingredient, followed by cooling; and a method in which the (A) ingredient, the (B) ingredient, the (C) ingredient, and the (E) ingredient are melted at a high temperature, which is mixed with the (D) ingredient, followed by cooling.

Selection of the oil component used in the present invention is not limited in particular; it can be selected from those commonly used in cosmetics within a range that does not adversely affect the stability. Preferable oil components include hydrocarbon oil components, polar oils such as ester oils, silicone oils, and liquid fats and oils.

Examples of the hydrocarbon oil that can be used include liquid paraffin, squalane, squalene, paraffin, isoparaffin, and ceresin.

Examples of the silicone oils include chain-like silicones such as dimethylpolysiloxane, methylphenyl polysiloxane, and methyl hydrogen polysiloxane; ring silicones such as octamethylcyclotetrasiloxane, decamethylcyclopentasiloxane, and dodecamethylcyclohexasiloxane; silicone resins forming a three-dimensional network structure, and silicone rubbers.

Examples of the polar oils such as ester oils that can be used include pentaerythrityl tetraethylhexanoate, cetyl ethylhexanoate, jojoba oil, di-(phytosteryl/octyldodecyl) lauroyl glutamate, triisostearin, glyceryl diisostearate, trimethylhexanoin, (phytosteryl/behenyl) dimer dilinoleate, (phytosteryl/isostearyl/cetyl/stearyl/behenyl) dimer dilinoleate, isopropyl palmitate, phytosteryl macadamia nut fatty acid ester, pentaerythrityl tetra (behenate/benzoate/ethylhexanoate), ethylhexyl palmitate, myristyl myristate, and tripropylene glycol dipivalate.

Examples of the liquid fats and oils include linseed oil, camellia oil, macadamia nut oil, corn oil, mink oil, olive oil, avocado oil, sasanqua oil, castor oil, safflower oil, sunflower oil, almond oil, rapeseed oil, sesame oil, soybean oil, peanut oil, triglycerin, glycerin trioctanoate, and glycerin triisopalmitate.

### <Blend ratio>

In the present invention, the blend ratio of the oil component is not limited in particular; a preferable blend ratio is about 5-30 wt% relative to the total amount of the emulsified composition. If it is less than 5 wt%, then the usefulness of the emulsified composition on the skin may not be manifested sufficiently; on the other hand, if it is over 30 wt%, then instability due to coalescing oil particles and such is a concern and the sensation during use tends to be oily.

For the (E) ingredient, one, two or more types can be used concurrently.

### <Viscosity reduction prevention and water separation of the emulsified composition >

The emulsified composition used in the present invention can prevent a reduction in its viscosity over time. Furthermore, it does not show separation of water, manifesting good stability. The reason for this is believed to be as follows.

That is, in the *α*-gel, water can exist in two types of places. Firstly, the *α*-gel can take in water between the hydrophilic groups of the bimolecular membrane. The distance between the hydrophilic groups of the bimolecular membrane is about several to several tens nm. This water between the hydrophilic groups is considered to be taken in at equilibrium with no possibility of separation even after long term storage. Secondly, water can be retained in the matrix of the *α*-gel. Alpha-gel forms fine matrices and is able to pool and hold water in the matrices. These matrices are believed to change their state depending on the coexisting ingredients and such; it is believed that, depending on conditions, water can be separated and cause instability.

Various ingredients that are usually used in cosmetics can be blended into the emulsified composition of the present invention as long as the stability of the emulsified composition is not adversely affected. Examples of such ingredients include, but are not limited to, monovalent alcohols, polyvalent alcohols, water soluble polymers, sequestering agents, antioxidants, perfumes, pigments, and powders.

The emulsified composition used in the present invention is manufactured by using a conventional method in the form of skin care cosmetics such as emulsions, moisture retaining creams, massage creams, cleansing creams, and essences.

### EXAMPLES

The emulsified cosmetic used in the present invention is described in detail below by referring to Examples.

### "State"

The state of each sample was observed visually.

### "Viscosity and pH"

The viscosity of the emulsified composition was measured at 30°C using a B-type viscometer (rotor number 3, rotor speed 12 rpm).

The pH of the emulsified composition was measured with a pH meter (Horiba pH meter F-13).

### "Test of viscosity stability over time"

Samples (emulsion and cream of the oil-in-water emulsified composition) obtained in Examples and Comparative examples were used to measure viscosity changes (a B-type viscometer at 30°C) after they were stored at -5°C, 0°C, RT, 37°C, and 50°C for one day and one month after preparation.

### <Criteria of stability>

### (Viscosity change after one month of storage)

○ : The value obtained by dividing the sample viscosity after storage by the sample viscosity right after preparation is 0.9 or more and less than 1. 1.

Δ : The value obtained by dividing the sample viscosity after storage by the sample viscosity right after preparation is 0.7 or more and less than 0.9, or 1.1 or more and less than 1.3.

× : The value obtained by dividing the sample viscosity after storage by the sample viscosity right after preparation is less than 0.7 or more than 1. 3.

○ and Δ in the aforementioned criteria were deemed to have passed the test and × was deemed to have failed the test.

### <Test of viscosity stability over time>

Formulations shown in Tables 1-6 were used to prepare emulsions or creams of the oil-in-water emulsified compositions, which were left alone at each temperature and investigated for viscosity changes over time.

### "Test 1: The effect of the presence or absence of a salt (ascorbic acid glucoside, sodium chloride) on the viscosity"

Ascorbic acid glucoside and sodium chloride, which are supposed to have an effect on the viscosity reduction of *α*-gel (Patent Document 1), were investigated for the effect of their addition on the viscosity of the emulsions.

### <Sample preparation method>

Emulsions were prepared as follows according to the formulations in Table 1.
1: Ingredients 1-8 are heated and mixed, and dissolved at 80°C ±2°C.
2: Ingredients 9-11 are wetted at room temperature.
3: Ingredients 12-16 are dissolved at 90-95°C.
4: 1 and 2 are mixed while being stirred.
5: With continuing stirring, 3 is added to 4 and mixed.
6: With continuing stirring, ingredients 17 and 18 are added to 5 and mixed.

Emulsion (oil-in-water emulsified composition)

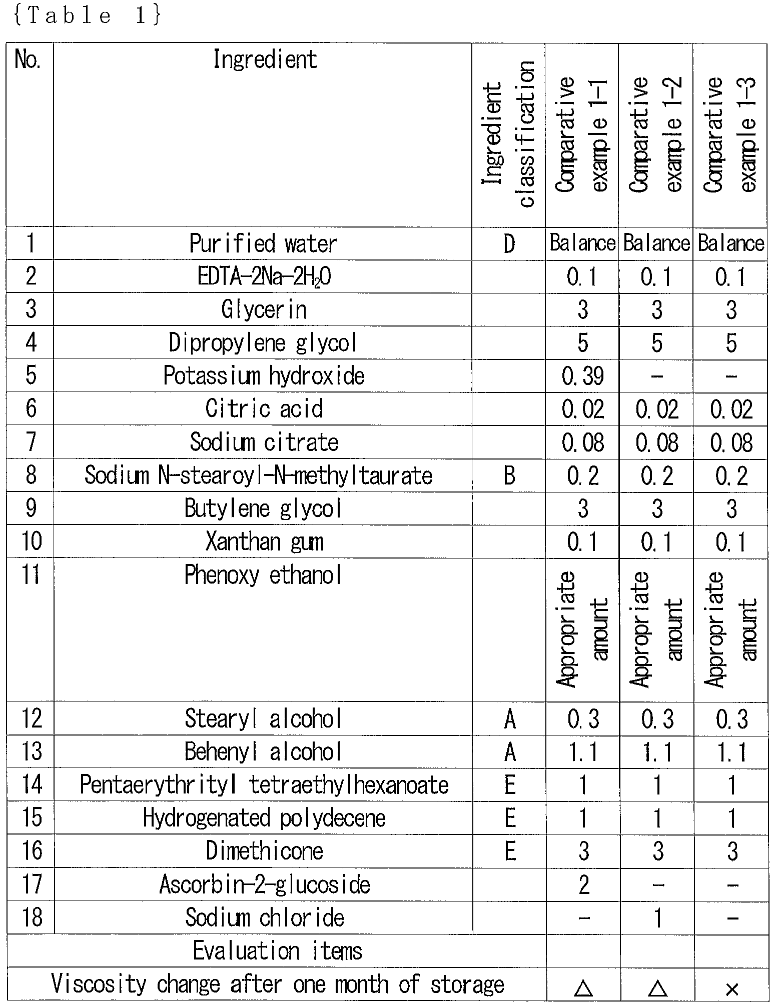

### <The results in Table 1 and discussions>

The viscosity reduction was suppressed by adding salts such as ascorbin-2-glucoside (in the potassium salt form in the formulation) and sodium chloride (Comparative example 1-1 and Comparative example 1-2); however, the sample to which these salts were not added (Comparative example 1-3) exhibited a reduction in viscosity.

Therefore, *α*-gel containing a higher aliphatic alcohol and an anionic surfactant such as sodium N-stearoyl-N-methyltaurate exhibits a reduction in viscosity when salts such as ascorbin-2-glucosie or sodium chloride are not added.

### "Test 2A and 2B: The effect of the addition of the cationic surfactant on the viscosity"

### <Sample preparation method>

Emulsions were prepared as follows according to the formulations in Table 2A.
1: Ingredients 1-7 are heated and mixed, and dissolved at 80°C ± 2°C.
2: Ingredients 8-10 are wetted at room temperature.
3: Ingredients 11-15 are dissolved at 90-95°C.
4: 1 and 2 are mixed while being stirred.
5: With continuing stirring, 3 is added to 4 and mixed.

Emulsion (oil-in-water emulsified composition)

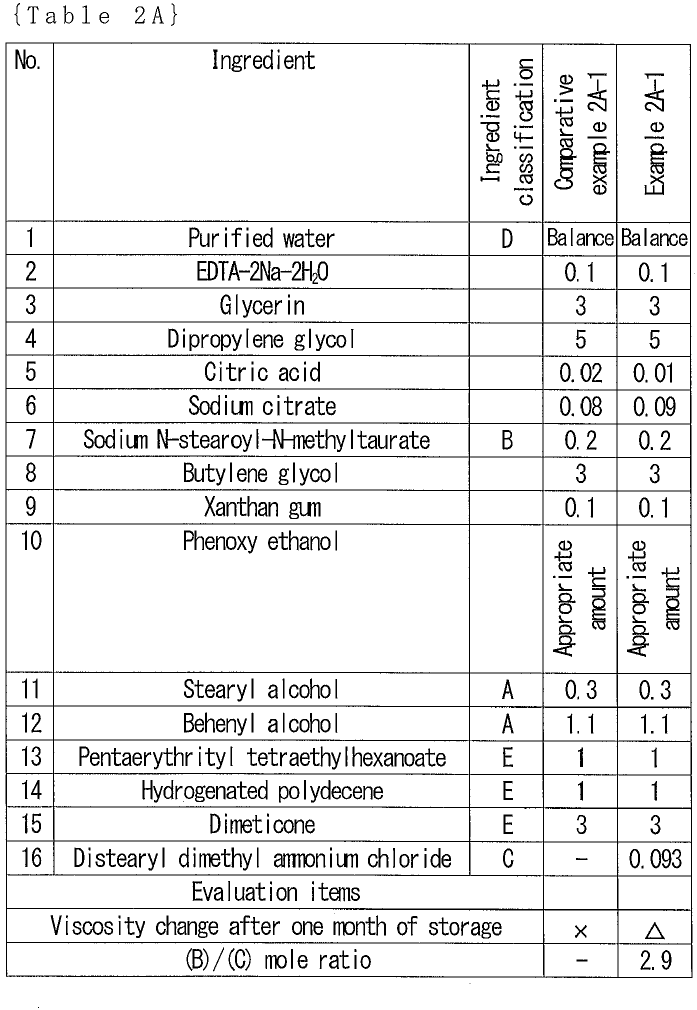

### <Sample preparation method>

Emulsions were prepared as follows according to the formulations in Table 2B.
1: Ingredients 1-8 are heated and mixed, and dissolved at 80°C ± 2°C.
2: Ingredients 9-11 are wetted at room temperature.
3: Ingredients 12-16 are dissolved at 90-95°C.
4: 1 and 2 are mixed while being stirred.
5: With continuing stirring, 3 is added to 4 and mixed.

Emulsion (oil-in-water emulsified composition)

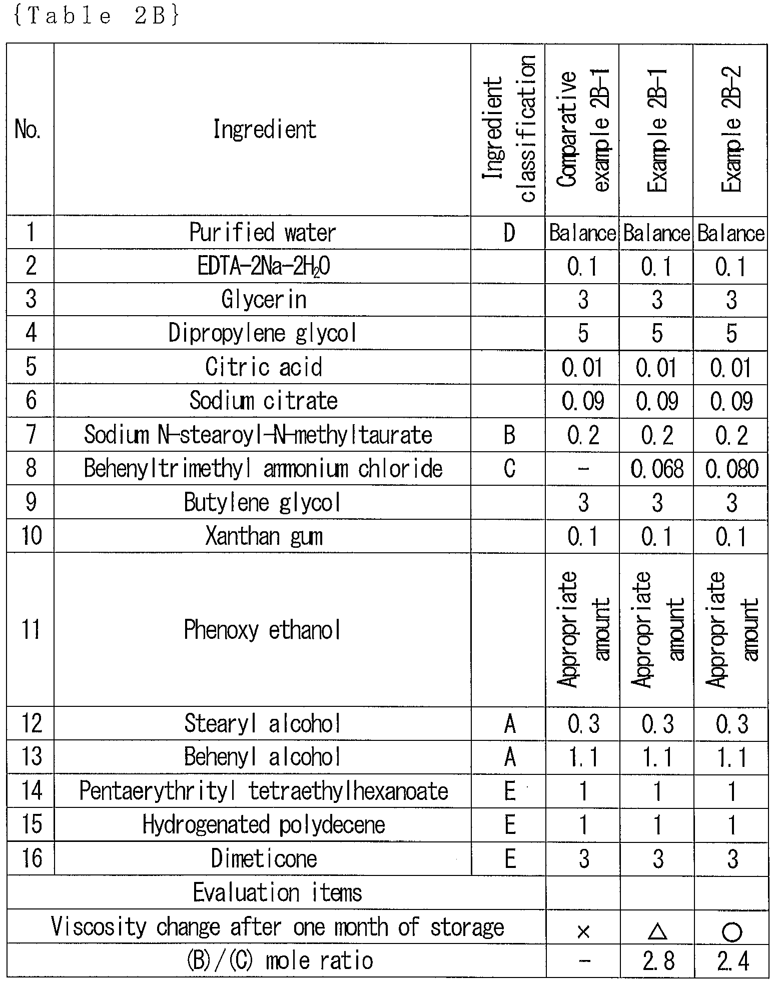

### <The results in Table 2A and 2B and discussion>

In Table 2A, Comparative example 2A-1 exhibited a reduction in viscosity due to the absence of the (C) ingredient, Distearyl dimethyl ammonium chloride. However, Example 2A-1, to which the (C) ingredient, distearyl dimethyl ammonium chloride, was added, exhibited a suppressed reduction in viscosity.

In Table 2B, Comparative example 2B-1 exhibited a reduction in viscosity due to the absence of the (C) ingredient, behenyl trimethyl ammonium chloride. However, Examples 2B-1 and 2B-2, to which the (C) ingredient, behenyl trimethyl ammonium chloride, was added, exhibited a suppressed reduction in viscosity.

"The effect of the addition (concentration) of the cationic surfactant on the viscosity"

### <Sample preparation method>

Emulsions were prepared as follows according to the formulations in Table 3.
1: Ingredients 1-7 are heated and mixed, and dissolved at 80°C ± 2°C.
2: Ingredients 8-10 are wetted at room temperature.
3: Ingredients 11-16 are dissolved at 90-95°C.
4: 1 and 2 are mixed while being stirred.
5: With continuing stirring, 3 is added to 4 and mixed.

Emulsion (oil-in-water emulsified composition)

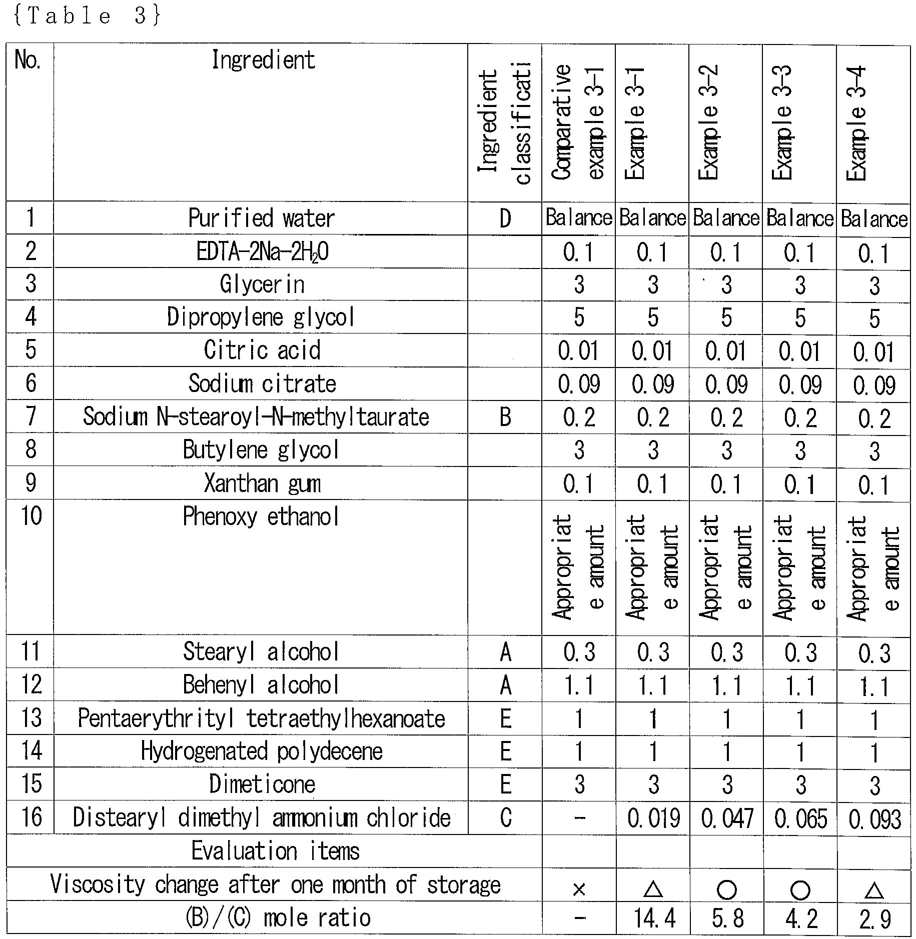

### <Sample preparation method>

Creams were prepared as follows according to the formulations in Table 4.
1: Ingredients 1-8 are heated and mixed, and dissolved at 80°C ± 2°C.
2: Ingredients 9 and 10 are wetted at room temperature.
3: Ingredients 11-17 are dissolved at 90-95°C.
4: 1 and 2 are mixed while being stirred.
5: With continuing stirring, 3 is added to 4 and mixed.

Cream (oil-in-water emulsified composition)

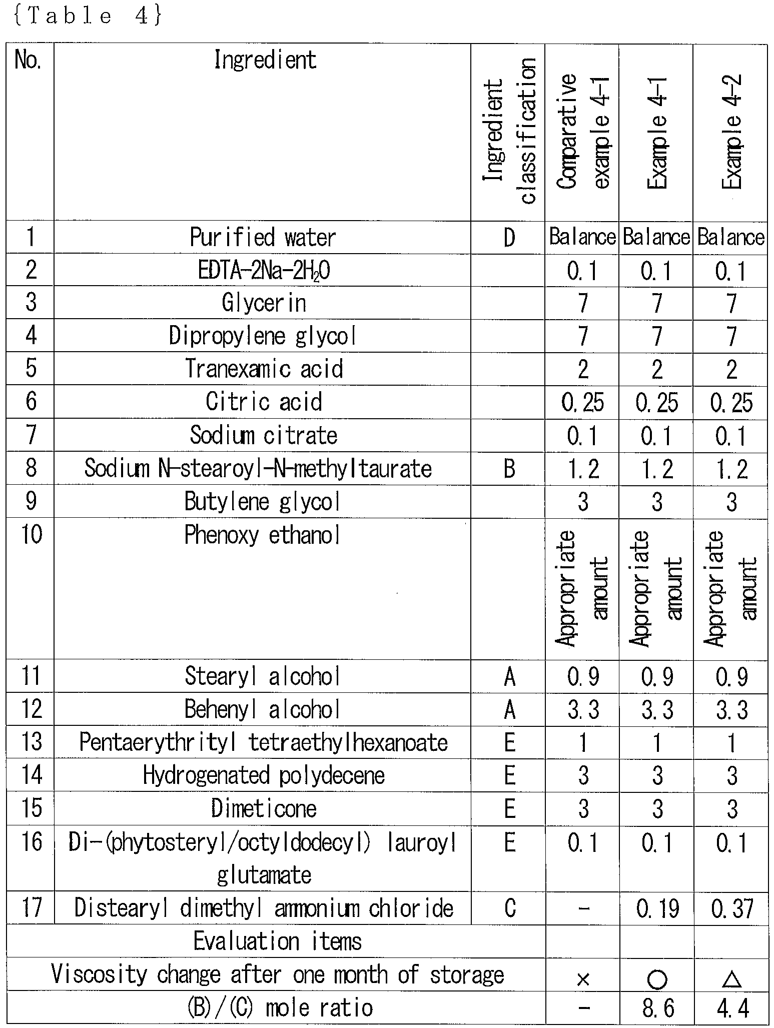

### <Reference Examples: Sample preparation method: Examples that use sodium N-stearoyl-L-glutamate for the (B) ingredient>

Emulsions were prepared as follows according to the formulations in Table 5.
1: Ingredients 1-7 are heated and mixed, and dissolved at 80°C ± 2°C.
2: Ingredients 8-10 are wetted at room temperature.
3: Ingredients 11-20 are dissolved at 90-95°C.
4: 1 and 2 are mixed while being stirred.
5: With continuing stirring, 3 is added to 4 and mixed.
6: With continuing stirring, ingredient 21 is added to 5 and mixed.

Emulsion (oil-in-water emulsified composition)

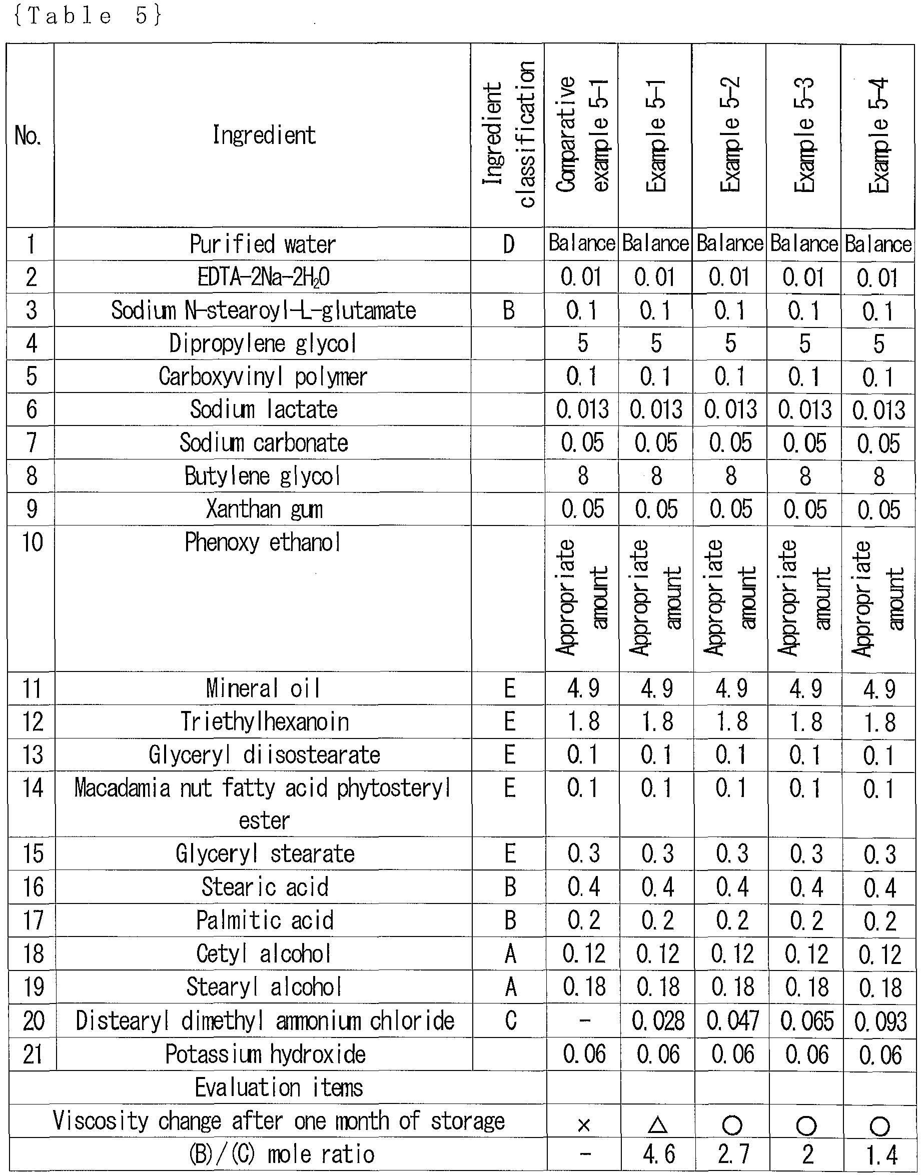

### <The results in Tables 3, 4, and 5 and discussion>

The aforementioned results indicate that the viscosity reduction of the emulsified composition of the present invention was suppressed when the blend ratio of the (B) ingredient was 1-15 times the blend ratio of the (C) ingredient in terms of the mole ratio.

### "Test 6. The effect of the pH on the viscosity of the emulsified composition"

### <Test of pH dependency of viscosity stability>

Emulsions of the oil-in-water emulsified composition were prepared based on the formulations shown in Table 6 and the viscosity changes at each pH were investigated.

### <Sample preparation method>

Emulsions were prepared as follows according to the formulations in Table 6.
1: Ingredients 1-7 are heated and mixed, and dissolved at 80°C ± 2°C.
2: Ingredients 8-10 are wetted at room temperature.
3: Ingredients 11-16 are dissolved at 90-95°C.
4: 1 and 2 are mixed while being stirred.
5: With continuing stirring, 3 is added to 4 and mixed.
6: With continuing stirring, ingredient 17 is added to 5 and mixed.

Emulsion (oil-in-water emulsified composition)

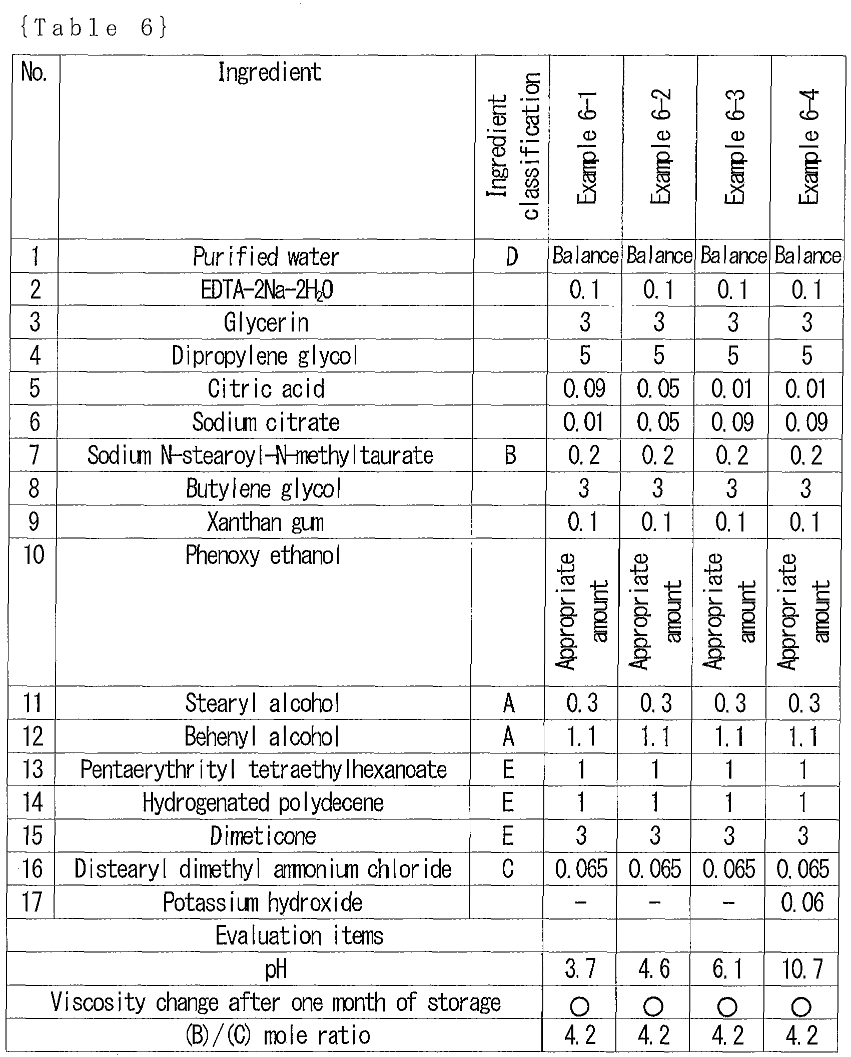

### <The results in Table 6 and discussion>

The emulsified composition system of the present invention exhibited no change in the effect of suppressing the viscosity reduction when the pH was changed in the range of 3-11. Therefore, the invention of the present application exhibits quite satisfactory viscosity stability over varying pH.

Other formulation examples of the present invention are shown below. Each formulation example is an emulsion or a cream that is superior in terms of the viscosity stability over time and in usability.

### "Formulation example 1: Emulsion"

| Ingredient | wt% |
|---|---|
| 1. Purified water (D ingredient) | Balance |
| 2. EDTA-2Na-2H₂O | 0.1 |
| 3. Citric acid | 0.01 |
| 4. Sodium citrate | 0.09 |
| 5. Sodium N-stearoyl-N-methyltaurate | 0.2 |
| (B ingredient, mole ratio of B ingredient/C ingredient : 5.8) | |
| 6. Distearyl dimethyl ammonium chloride (C ingredient) | 0.047 |
| 7. Stearyl alcohol (A ingredient) | 0.3 |
| 8. Behenyl alcohol (A ingredient) | 1.1 |
| 9. Pentaerythrityl tetraethylhexanoate (E ingredient) | 1.0 |
| 10. Dimethicone (E ingredient) | 3.0 |
| 11. Hydrogenated polydecene (E ingredient) | 1.0 |
| 12. Ethanol | 4.0 |
| 13. Xanthan gum | 0.1 |

### <Preparation method>

1: Ingredients 1-5 are heated and mixed, and dissolved at 80°C ± 2°C.
2: Ingredients 6-11 are dissolved at 90-95°C.
3: Ingredients 12-13 are wetted at room temperature.
4: 1 and 2 are mixed while being stirred. 5: With continuing stirring, 3 is added and mixed.

### "Formulation example 2: Cream"

| Ingredient | wt% |
|---|---|
| 1. Purified water (D ingredient) | Balance |
| 2. EDTA-2Na-2H₂O | 0.1 |
| 3. Glycerin | 7.0 |
| 4. Dipropylene glycol | 7.0 |
| 5. Butylene glycol | 3.0 |
| 6. Tranexamic acid | 2.0 |
| 7. Citric acid | 0.25 |
| 8. Sodium citrate | 0.1 |
| 9. Sodium N-stearoyl-N-methyltaurate | 1.2 |
| (B ingredient, mole ratio of B ingredient/C ingredient : 4.4) | |
| 10. Distearyl dimethyl ammonium chloride (C ingredient) | 0.37 |
| 11. Stearyl alcohol (A ingredient) | 0.9 |
| 12. Behenyl alcohol (A ingredient) | 3.3 |
| 13. Pentaerythrityl tetraethylhexanoate (E ingredient) | 1.0 |
| 14. Dimethicone (E ingredient) | 3.0 |
| 15. Hydrogenated polydecene (E ingredient) | 3.0 |
| 16. Di-(phytosteryl/octyldodecyl) lauroyl glutamate (E ingredient) | 0.1 |
| 17. Preservative | Appropriate amount |

### <Preparation method>

1: Ingredients 1-9 are heated and mixed, and dissolved at 80°C ± 2°C.
2: Ingredients 10-16 are dissolved at 90-95°C.
3: 1 and 2 are mixed while being stirred.
4: With continuing stirring, 17 is added and mixed.

### "Formulation example 3: Emulsion"

| Ingredient | wt% |
|---|---|
| 1. Purified water (D ingredient) | Balance |
| 2. EDTA-2Na-2H₂O | 0.1 |
| 3. Citric acid | 0.01 |
| 4. Sodium citrate | 0.09 |
| 5. Sodium N-stearoyl-N-methyltaurate | 0.2 |
| (B ingredient, mole ratio of B ingredient/C ingredient : 5.8) | |
| 6. Distearyl dimethyl ammonium chloride (C ingredient) | 0.047 |
| 7. Lauryl alcohol (A ingredient) | 0.3 |
| 8. Myristyl alcohol (A ingredient) | 1.1 |
| 9. Pentaerythrityl tetraethylhexanoate (E ingredient) | 1.0 |
| 10. Dimethicone (E ingredient) | 3.0 |
| 11. Hydrogenated polydecene (E ingredient) | 1.0 |
| 12. Ethanol | 4.0 |
| 13. Xanthan gum | 0.1 |

### <Preparation method>

1: Ingredients 1-5 are heated and mixed, and dissolved at 80°C ± 2°C.
2: Ingredients 6-11 are dissolved at 90-95°C.
3: Ingredients 12 and 13 are wetted at room temperature.
4: 1 and 2 are mixed while being stirred.
5: With continuing stirring, 3 is added and mixed.

### "Formulation example 4: Emulsion"

| Ingredient | wt% |
|---|---|
| 1. Purified water (D ingredient) | Balance |
| 2. EDTA-2Na-2H₂O | 0.1 |
| 3. Citric acid | 0.01 |
| 4. Sodium citrate | 0.09 |
| 5. Sodium N-stearoyl-N-methyltaurate | 0.2 |
| (B ingredient, mole ratio of B ingredient/C ingredient : 5.8) | |
| 6. Distearyl dimethyl ammonium chloride (C ingredient) | 0.047 |
| 7. Batyl alcohol (A ingredient) | 0.3 |
| 8. Stearyl alcohol (A ingredient) | 1.1 |
| 9. Pentaerythrityl tetraethylhexanoate (E ingredient) | 1.0 |
| 10. Dimethicone (E ingredient) | 3.0 |
| 11. Hydrogenated polydecene (E ingredient) | 1.0 |
| 12. Ethanol | 4.0 |
| 13. Xanthan gum | 0.1 |

### <Preparation method>

1: Ingredients 1-5 are heated and mixed, and dissolved at 80°C ± 2°C.
2: Ingredients 6-11 are dissolved at 90-95°C.
3: Ingredients 12 and 13 are wetted at room temperature.
4: 1 and 2 are mixed while being stirred.
5: With continuing stirring, 3 is added and mixed.

### INDUSTRIAL APPLICABILITY

The present invention a non-therapeutic use of an emulsified composition as a skin care cosmetic wherein the viscosity of the emulsified composition does not decrease over time even when salts such as ascorbic acid 2-glucoside are not added and thus the stability over time is excellent; this is accomplished by using a cationic surfactant in an emulsified composition having specific ingredients involving *α*-gel.

The stability of an emulsified composition over time is a very important factor. Therefore, in the field of emulsified compositions involving *α*-gel composed of specific ingredients, the present invention exhibits an absence of viscosity reduction over time, indicating superior stability over time, and its effect is manifested in a wide pH range, unaffected by the pH, therefore it has a very high industrial application potential as an emulsified composition or an emulsified cosmetic.

## Claims

1. Non-therapeutic use of an emulsified cosmetic as a skin care cosmetic, wherein the emulsified composition comprises the following (A)-(E) ingredients wherein the blend ratio of the (B) ingredient is 1-15 times the blend ratio of the (C) ingredient in terms of mole ratio, the pH of said emulsified composition is 3-11, and said emulsified composition is an emulsified cosmetic,
wherein
the (A) ingredient is a higher aliphatic alcohol,
the (B) ingredient is an anionic surfactant,
the (C) ingredient is a cationic surfactant,
the (D) ingredient is water,
the (E) ingredient is an oil component,
**characterized in that**:
said (B) ingredient comprises a long chain acyl sulfonate anionic surfactant represented by the general formula (I),
general formula (I)
R₁CO-a-(CH₂)ₙSO₃M₁ (I)
wherein in formula (I), R₁CO- denotes a saturated or unsaturated fatty acid residue (acyl group) having 10-22 carbon atoms on average; a denotes -NR₂-, wherein R₂ denotes a hydrogen atom or an alkyl group having 1-3 carbon atoms; M₁ denotes a hydrogen atom, alkali metal, alkali earth metal, ammonium, or organic amine; n denotes an integer 1-3,
said (C) ingredient, the cationic surfactant, comprises a dialkyl quaternary ammonium salt and/or monoalkyl quaternary ammonium salt.

2. Non-therapeutic use of the emulsified cosmetic of claim 1 wherein said (B) ingredient is sodium N-stearoyl-N-methyltaurate.

## Patentansprüche

1. Nichttherapeutische Verwendung eines emulgierten Kosmetikums als ein Hautpflegekosmetikum, wobei die emulgierte Zusammensetzung die folgenden (A)-(E)-Bestandteile umfasst, wobei das Mischungsverhältnis des (B)-Bestandteils das 1-15-fache des Mischungsverhältnisses des (C)-Bestandteils, ausgedrückt als Mol-Verhältnis, beträgt, der pH der emulgierten Zusammensetzung 3-11 beträgt, und die emulgierte Zusammensetzung ein emulgiertes Kosmetikum ist, wobei
der (A)-Bestandteil ein höherer aliphatischer Alkohol ist,
der (B)-Bestandteil ein anionisches Tensid ist,
der (C)-Bestandteil ein kationisches Tensid ist,
der (D)-Bestandteil Wasser ist,
der (E)-Bestandteil eine Ölkomponente ist,
**dadurch gekennzeichnet, dass**
der (B)-Bestandteil ein durch die allgemeine Formel (I) wiedergegebenes, anionisches, langkettiges Acylsulfonat-Tensid umfasst, allgemeine Formel (I)
R¹C-a-(CH₂)ₙSO₃M₁ (I)
worin in der Formel (I), R₁CO- einen gesättigten oder ungesättigten Fettsäurerest (Acylgruppe) mit im Durchschnitt 10-22 Kohlenstoffatomen bedeutet; a -NR₂- bedeutet, worin R₂ ein Wasserstoffatom oder eine Alkylgruppe mit 1-3 Kohlenstoffatomen bedeutet; M₁ ein Wasserstoffatom, Alkalimetall, Erdalkalimetall, Ammonium oder organisches Amin bedeutet; n eine ganze Zahl 1-3 bedeutet,
der in (C)-Bestandteil, das kationische Tensid, ein quaternäres Dialkylammoniumsalz und/oder quaternäres Monoalkylammoniumsalz umfasst.

2. Nichttherapeutische Verwendung des emulgierten Kosmetikums nach Anspruch 1, wobei der (B)-Bestandteil Natrium-N-stearoyl-N-methyltaurat ist.

## Revendications

1. Usage non-thérapeutique d'un cosmétique émulsifié en tant que cosmétique pour soin de la peau, dans lequel la composition émulsifiée comprend les ingrédients (A) - (E) suivants, dans lequel le rapport de mélange de l'ingrédient (B) est 1-15 fois le rapport de mélange de l'ingrédient (C) en termes de rapport molaire, le pH de ladite composition émulsifiée est 3-11, et ladite composition émulsifiée est un cosmétique émulsifié, dans lequel
l'ingrédient (A) est un alcool aliphatique supérieur,
l'ingrédient (B) est un surfactant anionique,
l'ingrédient (C) est un surfactant cationique,
l'ingrédient (D) est de l'eau,
l'ingrédient (E) est un composant d'huile,
**caractérisé en ce, que** :
ledit ingrédient (B) est un tensioactif acylsulfonate anionique à longue chaîne représenté par la formule générale (I) suivante:
R₁CO-a-(CH₂)ₙSO₃M₁ (I),
dans lequel dans la formule (I), R₁CO- désigne un résidu d'acide gras saturé ou insaturé (groupe acyle) ayant en moyenne de 10 à 22 atomes de carbone; a désigne -NR₂-, où R₂ représente un atome d'hydrogène ou un groupe alkyle ayant de 1 à 3 atomes de carbone ; M1 désigne un atome d'hydrogène, un métal alcalin, un métal alcalino-terreux, l'ammonium ou une amine organique; n représente un nombre entier de 1 à 3,
ledit ingrédient (C), le surfactant cationique, comprend un sel de dialkylammonium quaternaire et / ou un sel de monoalkylammonium quaternaire.

2. Usage non-thérapeutique du cosmétique émulsifié selon la revendication 1, dans lequel ledit ingrédient (B) est un N-stéaroyl-N-méthyltaurate.
